# EUROPEAN PATENT APPLICATION

(11) **EP 4 389 013 A1**
(43) Date of publication of application: **26.06.2024**
(21) Application number: 22872990.1
(22) Date of filing: 22.09.2022
(51) Int. Cl.: A61B 8/00

(54) **ULTRASONIC DIAGNOSTIC DEVICE**

(30) Priority: 27.09.2021 JP 2021157068
(71) Applicant: CANON KABUSHIKI KAISHA, Tokyo 146-8501 (JP)
(72) Inventor: NIWAMAE Yuki, Tokyo 146-8501 (JP); TANIMOTO Sakiko, Tokyo 146-8501 (JP); YAMAMOTO Takahiro, Tokyo 146-8501 (JP); SHINATA Satoshi, Tokyo 146-8501 (JP); HOKARI Yusuke, Tokyo 146-8501 (JP); KAWASHIMA Shosaku, Tokyo 146-8501 (JP); HIRANO Yoshinori, Tokyo 146-8501 (JP); NAKAYAMA Akiya, Tokyo 146-8501 (JP)
(74) Representative: WESER & Kollegen Patentanwälte PartmbB
(86) International application number: PCT/JP2022/035399
(87) International publication number: WO 2023/048239

(57) **Abstract**

An ultrasonic diagnostic apparatus with improved operability is provided. An ultrasonic diagnostic apparatus according to the present invention includes an ultrasonic probe (142, 144) that transmits and receives ultrasonic waves to and from a subject, an accommodation portion that accommodates the ultrasonic probe (142, 144), a display unit (122) that displays an ultrasonic image based on the ultrasonic waves transmitted and received by using the ultrasonic probe (142, 144), an operating portion (124) that has a plurality of input elements and that is used to input operating information of an operator, a bed (106) for mounting the subject, and a supporting portion (104) that is provided upright on a floor or a ceiling and that supports the operating portion (124), the bed (106), and the accommodation portion (140).

## Description

### Technical Field

The present invention relates to an ultrasonic diagnostic apparatus that generates an ultrasonic image by transmitting and receiving ultrasonic waves to and from a subject.

### Background Art

In an existing ultrasonic diagnostic apparatus, an ultrasonic probe is brought into contact with a subject mounted on a bed configured separately from an apparatus main body of the ultrasonic diagnostic apparatus, and an ultrasonic signal received by the ultrasonic probe is processed in the apparatus main body to generate an ultrasonic image. Then, a display unit of the ultrasonic diagnostic apparatus displays the ultrasonic image, a measurement result, and the like. The ultrasonic diagnostic apparatus that allows an operator to place the display unit at a position where the operator can easily see the ultrasonic image is disclosed (for example, PTL 1).

### Citation List

### Patent Literature

PTL 1: Japanese Patent Laid-Open No. 2010-046374

### Summary of Invention

### Technical Problem

In an ultrasonic diagnostic apparatus described in PTL 1, an operating portion for an operator to operate and an accommodation portion that accommodates an ultrasonic probe are installed in the ultrasonic diagnostic apparatus separately provided from a bed on which a subject is mounted. The operator operates the operating portion while bringing the ultrasonic probe into contact with the subject mounted on the bed, so the subject can be photographed in an unnatural position.

Therefore, it is an object of the present invention to provide an ultrasonic diagnostic apparatus with improved operability.

### Solution to Problem

To solve the above-problem, an ultrasonic diagnostic apparatus according to the present invention includes an ultrasonic probe that transmits and receives ultrasonic waves to and from a subject, an accommodation portion that accommodates the ultrasonic probe, a display unit that displays an ultrasonic image based on the ultrasonic waves transmitted and received by using the ultrasonic probe, an operating portion that has a plurality of input elements and that is used to input operating information of an operator, a bed for mounting the subject, and a supporting portion that is installed on a floor or a ceiling and that supports the operating portion, the bed, and the accommodation portion.

### Advantageous Effects of Invention

According to the present invention, it is possible to improve operability of the ultrasonic diagnostic apparatus.

### Brief Description of Drawings

[Fig. 1] Fig. 1 is a perspective view that shows the configuration of an ultrasonic diagnostic apparatus according to the present invention.
[Fig. 2] Fig. 2 is a top view that shows the configuration of the ultrasonic diagnostic apparatus according to the present invention.
[Fig. 3] Fig. 3 is a perspective view that shows the configuration of the ultrasonic diagnostic apparatus according to the present invention.
[Fig. 4] Fig. 4 is a block diagram that shows the configuration of an apparatus main body of the ultrasonic diagnostic apparatus according to the present invention.
[Fig. 5] Fig. 5 is a view that shows up and down drive of components of the ultrasonic diagnostic apparatus according to the present invention.
[Fig. 6] Fig. 6 is a view that shows up and down drive of the components of the ultrasonic diagnostic apparatus according to the present invention.
[Fig. 7A] Fig. 7A is a view that shows up and down drive of the components of the ultrasonic diagnostic apparatus according to the present invention.
[Fig. 7B] Fig. 7B is a view that shows up and down drive of the components of the ultrasonic diagnostic apparatus according to the present invention.
[Fig. 8] Fig. 8 is a view that shows the configuration of a frame portion of the ultrasonic diagnostic apparatus according to the present invention.
[Fig. 9] Fig. 9 is a view that shows the configuration of the frame portion of the ultrasonic diagnostic apparatus according to the present invention.
[Fig. 10] Fig. 10 is a view that shows the configuration of an operating portion of the ultrasonic diagnostic apparatus according to the present invention.
[Fig. 11] Fig. 11 is a view that shows a modification of a bed of the ultrasonic diagnostic apparatus according to the present invention.
[Fig. 12] Fig. 12 is a view that shows a modification of components (the bed, the operating portion, and the like) of the ultrasonic diagnostic apparatus according to the present invention.

### Description of Embodiments

An ultrasonic diagnostic apparatus according to the present invention includes an ultrasonic probe that is brought into contact with a subject and that transmits and receives ultrasonic waves, an operating portion that has a plurality of input elements and that is used to input operating information of an operator, a bed for mounting the subject, a display unit that displays an ultrasonic image generated by processing an ultrasonic signal received by the ultrasonic probe, a measurement result, and the like.

The ultrasonic diagnostic apparatus according to the present invention includes a supporting portion that supports components of the ultrasonic diagnostic apparatus. The supporting portion supports the operating portion and the bed such that the operating portion and the bed are movable up and down and supports the display unit and the bed such that the display unit and the bed are movable up and down. The ultrasonic diagnostic apparatus according to the present invention includes an accommodation portion that accommodates a plurality of the ultrasonic probes. The supporting portion supports the accommodation portion.

The ultrasonic diagnostic apparatus according to the present invention may be translated into an ultrasonic diagnostic apparatus with a bed, an ultrasonic diagnostic system, an ultrasonic photographing system, or the like.

Hereinafter, a preferred embodiment of the present invention will be described with reference to the attached drawings.

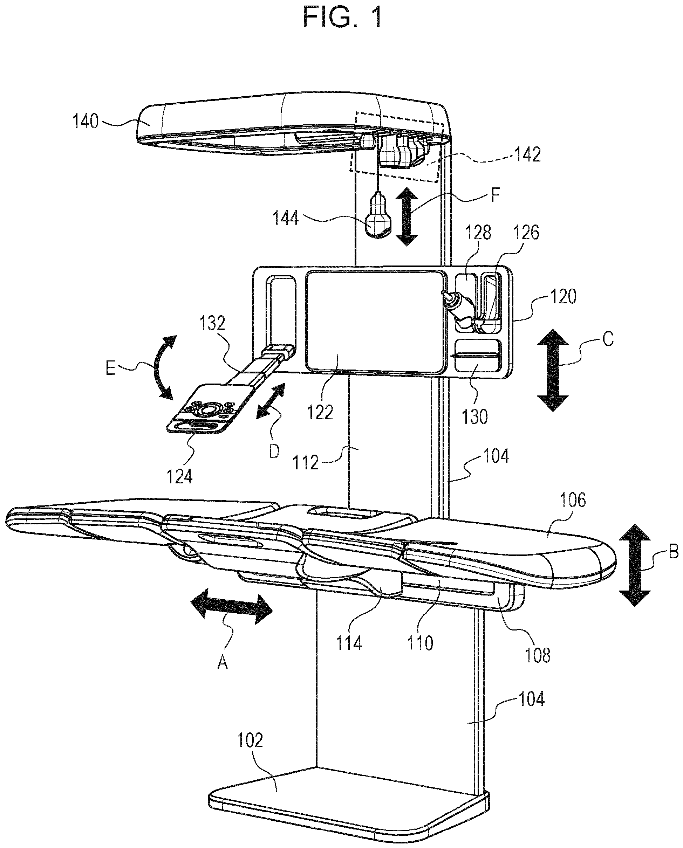

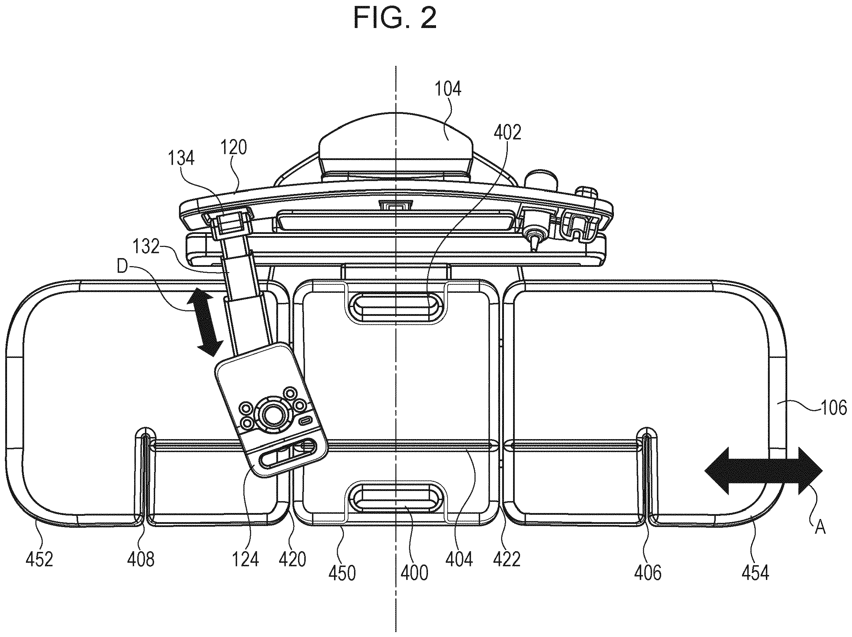

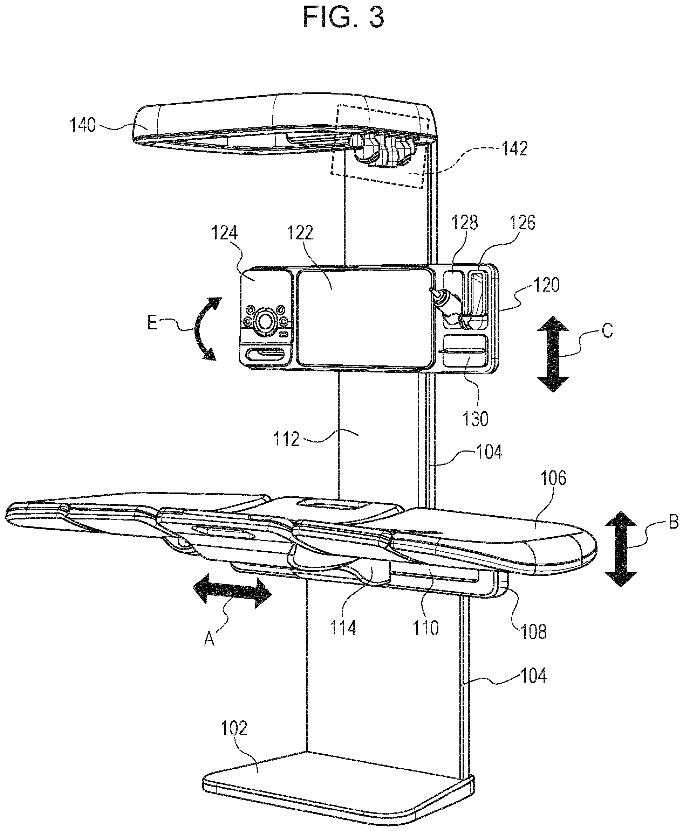

Figs. 1 to 3 show the configuration of the ultrasonic diagnostic apparatus according to the present invention. Figs. 1 and 3 are perspective views of the ultrasonic diagnostic apparatus. Fig. 2 is a top view of the ultrasonic diagnostic apparatus.

The ultrasonic diagnostic apparatus includes a base portion 102 that supports the ultrasonic diagnostic apparatus on a floor. The base portion 102 is a member that is in contact with the floor. The base portion 102 is fixed to the floor by a screw or the like. When fixing of the base portion 102 to the floor is released, an operator is able to move the ultrasonic diagnostic apparatus. A configuration in which the base portion 102 is installed on the floor is shown. Alternatively, a configuration in which another base portion (not shown) is fixed to a ceiling and the ultrasonic diagnostic apparatus is suspended from the ceiling may be applied.

The ultrasonic diagnostic apparatus includes a supporting portion 104 that is provided upright on the floor or the ceiling and that supports the components of the ultrasonic diagnostic apparatus. The supporting portion 104 is integrated with the base portion 102. The supporting portion 104 is a member extending in an up and down direction (vertical direction) and may be translated into a mast. The supporting portion 104 disposes the components of the ultrasonic diagnostic apparatus in the up and down direction and supports the components.

The ultrasonic diagnostic apparatus includes a bed 106 for mounting a subject. The subject is mounted on the top surface of the bed 106. The supporting portion 104 supports the bed 106 such that the top surface of the bed 106 is horizontal. The bed 106 is supported by the supporting portion 104 through a slide 108 installed on the supporting portion 104. The slide 108 has a groove 110 in a horizontal direction. A bed support member 114 installed on the bottom surface of the bed 106 is fitted to the groove 110 of the slide 108. When the bed support member 114 of the bed 106 is moved in the horizontal direction with respect to the groove 110 of the slide 108, the bed 106 can slide in the horizontal direction (A direction) through the slide 108. The horizontal direction (A direction) is a longitudinal direction of the bed 106.

The bed 106 is supported by the supporting portion 104 through a coupling portion 112 installed on the supporting portion 104. Since the coupling portion 112 is supported so as to be movable in the up and down direction with respect to the supporting portion 104, the bed 106 can be moved in the up and down direction (B direction). In other words, the supporting portion 104 supports the bed 106 such that the bed 106 is movable in the up and down direction. Since the supporting portion 104 supports the bed 106 and the subject, from which a load is applied, the supporting portion 104 located on the lower side of the bed 106 is longer in perimeter and wider than the supporting portion 104 located on the upper side of the bed 106. In this way, the rigidity of the ultrasonic diagnostic apparatus is maintained by changing the perimeter of the mast portion 104.

The ultrasonic diagnostic apparatus includes a panel unit 120 including a plurality of components. The panel unit 120 includes a display unit 122 that displays an ultrasonic image, generated by processing an ultrasonic signal, a measurement result, and the like. The display unit 122 is installed on the panel unit 120 so as to be rotatable. The operator is able to set the display unit 122 to a horizontally oriented state and a vertically oriented state by rotating the display unit 122. The display unit 122 shown in Fig. 1 is in the horizontally oriented state. The operator is able to set the display unit 122 in the vertically oriented state by rotating the display unit 122 by 90 degrees. The display unit 122 allows to change the layout of the ultrasonic image and the measurement result in synchronization with the rotational angle of the display unit 122.

The panel unit 120 has an operating portion 124 for the operator to operate. The panel unit 120 includes an ultrasonic probe holder 126 for mounting an ultrasonic probe, a bottle accommodation portion 128 for accommodating a bottle for ultrasonic jelly, and a tissue paper box 130 for removing ultrasonic jelly stuck to the subject. The operating portion 124 is installed at one end (left side) of the panel unit 120. The ultrasonic probe holder 126, the bottle accommodation portion 128, and the tissue paper box 130 are installed at the other end (right side) of the panel unit 120. In the panel unit 120, the display unit 122 is installed between the operating portion 124 and the set of ultrasonic probe holder 126, bottle accommodation portion 128, and tissue paper box 130.

The ultrasonic probe holder 126 is a holding portion for temporarily putting the ultrasonic probe 144 being used for ultrasonic photographing. The bottom surface of the ultrasonic probe holder 126 projects from the panel unit 120. The bottom surface of the ultrasonic probe holder 126 is a curved surface. The ultrasonic probe holder 126 is capable of holding an ultrasonic transmitting and receiving face (head part) of the ultrasonic probe 144 with the bottom surface of the ultrasonic probe holder 126.

The bottle accommodation portion 128 has a function (jelly warmer function) of warming the bottle for ultrasonic jelly. The bottle accommodation portion 128 is capable of keeping the bottle for ultrasonic jelly at a predetermined temperature (for example, 35°C to 40°C).

In this way, the panel unit 120 is capable of holding necessary items at the time of using the ultrasonic diagnostic apparatus. Therefore, the operator is able to acquire necessary items by operating the operating portion of the ultrasonic diagnostic apparatus without changing his or her position.

The panel unit 120 is coupled to the bed 106 through the coupling portion 112. The coupling portion 112 is installed along the longitudinal direction of the supporting portion 104. The longitudinal direction of the coupling portion 112 is parallel to the longitudinal direction of the supporting portion 104. For example, a groove or a rail (not shown) is formed along the longitudinal direction of the supporting portion 104. Part of the coupling portion 112 is fitted to the groove or rail of the supporting portion 104, and the coupling portion 112 is installed on the supporting portion 104. Therefore, even when the load of the bed 106 and the like is applied to the coupling portion 112, the configuration as shown in Fig. 1 can be maintained.

The coupling portion 112 is installed on the supporting portion 104 so as to be movable in the up and down direction. Since the coupling portion 112 can be moved in the up and down direction with respect to the supporting portion 104, the panel unit 120 can be moved in the up and down direction (C direction). The display unit 122 and the operating portion 124, installed on the panel unit 120, can be moved in the up and down direction via the coupling portion 112.

The operating portion 124 is installed on the panel unit 120 through an arm 132. The arm 132 has such a structure that a plurality of oblong cylinder portions is inserted in each other. In other words, the arm 132 has a telescoping structure and allows to extend and contract in a longitudinal direction (D direction) of the arm 132. The position of the operating portion 124 can be changed by extending and contracting the arm 132. The arm 132 is installed on the panel unit 120 through a hinge (described later). The hinge serves as a pivot of the arm 132, so the arm 132 can be pivoted in a predetermined rotation direction (E direction). Therefore, the operator is able to fold the arm 132 through the hinge. The operator is able to accommodate the operating portion 124 in the panel unit 120 by folding the arm 132.

Here, the configuration in which the display unit 122 and the operating portion 124 are supported by the supporting portion 104 through the panel unit 120 has been described; however, the display unit 122 and the operating portion 124 may be directly supported by the supporting portion 104. In this case, the display unit 122 and the operating portion 124 each independently allow to move in the up and down direction.

The ultrasonic diagnostic apparatus includes a frame portion 140. The supporting portion 104 supports the frame portion 140. The frame portion 140 is supported at the topmost part of the supporting portion 104. The frame portion 140 is a square-shaped annular member. To reduce a feeling of pressure to the subject mounted on the bed 106, the central part of the frame portion 140 is hollow. The plurality of ultrasonic probes 142, 144 is installed on the frame portion 140. The frame portion 140 may be translated into an accommodation portion for the ultrasonic probes.

The accommodation portion for the ultrasonic probes is installed in the frame portion 140 such that, when the ultrasonic probe 144 used for photographing is drawn out (lowered) from the accommodation portion for cables of the ultrasonic probes, the ultrasonic probe 144 and the operating portion 124 do not interfere (contact) with each other. Here, the ultrasonic probe 144 will be described as the ultrasonic probe used for photographing, and the description also applies to the other ultrasonic probes 142. Specifically, the accommodation portion for the ultrasonic probes is installed in the frame portion 140 at the right side when viewed from the front of the ultrasonic diagnostic apparatus. The ultrasonic probe 144 used for photographing lowers from the frame portion 140 at the right side of the panel unit 120 (near the ultrasonic probe holder 126 and the bottle accommodation portion 128). In this way, the accommodation portion for the ultrasonic probes is installed just above the ultrasonic probe holder 126 and the bottle accommodation portion 128. For this reason, the ultrasonic probe 144 lowers from the frame portion 140 to near the ultrasonic probe holder 126, so the ultrasonic probe 144 can be temporarily put on the ultrasonic probe holder 126.

On the other hand, the operating portion 124 is installed at the left side when viewed from the front of the ultrasonic diagnostic apparatus. Specifically, the operating portion 124 is installed at the left side of the panel unit 120. In this way, the accommodation portion for the ultrasonic probes is not installed in the frame portion 140 just above the operating portion 124. For this reason, even when the ultrasonic probe 144 used for photographing lowers from the frame portion 140, the ultrasonic probe 144 does not interfere (contact) with the operating portion 124.

As described above, the supporting portion 104 is provided upright on the floor or the ceiling and supports the components. Here, the supporting portion 104 supports the bed 106, the operating portion 124, and the frame portion 140 (the accommodation portion for the ultrasonic probes) so as to be spaced apart from each other in the up and down direction. The supporting portion 104 supports the bed 106, the display unit 122, and the frame portion 140 (the accommodation portion for the ultrasonic probes) so as to be spaced apart from each other in the up and down direction. The supporting portion 104 supports the components so as to be spaced apart from each other in the up and down direction separately in three areas, that is, a top part, a middle part, and a bottom part.

The supporting portion 104 supports the components such that the components extend in one direction (a direction toward the front, that is, a direction orthogonal to the A direction and the B direction). The supporting portion 104 has a so-called cantilever structure and supports the components with no external support other than the supporting portion 104. The bed 106, the operating portion 124, the display unit 122, and the frame portion 140 (the accommodation portion for the ultrasonic probes) are installed on the supporting portion 104 so as to extend in one direction. When the bed 106, the operating portion 124 or the display unit 122, and the frame portion 140 (the accommodation portion for the ultrasonic probes) are installed on the supporting portion 104 so as to be spaced apart from each other, the components have a layer structure.

Specifically, the bed 106 is supported at the bottom part of the supporting portion 104. The display unit 122 and the operating portion 124 are supported at the middle part of the supporting portion 104. The frame portion 140 (the accommodation portion for the ultrasonic probes) is supported at the top part of the supporting portion 104. The display unit 122 is installed above the bed 106, and the operating portion 124 is installed above the bed 106. In other words, the display unit 122 is installed at a position higher than the position where the bed 106 is installed. The operating portion 124 is installed at a position higher than the position where the bed 106 is installed.

The frame portion 140 is installed above the display unit 122, and the frame portion 140 is installed above the operating portion 124. In other words, the frame portion 140 is installed at a position higher than the position where the display unit 122 is installed. The frame portion 140 is installed at a position higher than the position where the operating portion 124 is installed.

The frame portion 140 (the accommodation portion for the ultrasonic probes) is installed just above the bed 106. Therefore, when the plurality of ultrasonic probes 142, 144 is lowered from the frame portion 140, the ultrasonic probes are disposed above the bed 106.

Since the display unit 122 is installed above the bed 106, the operator is able to check the state of the subject mounted on the bed 106, a contact point of the ultrasonic probe 144 used for photographing the subject, and the like and observe an ultrasonic image, a measurement result, and the like displayed on the display unit 122. Since the operating portion 124 is installed above the bed 106, the operator is able to check the state of the subject, an installation point of the ultrasonic probe 144 used for photographing, and the like and operate the operating portion 124. In the ultrasonic diagnostic apparatus according to the present invention, it is assumed that the operator operates while standing near the center of the bed 106 in the longitudinal direction. For this reason, the components, that is, the display unit 122, the operating portion 124, the ultrasonic probe 144, and the bed 106, can be installed toward the front of the operator. Therefore, the operator is able to perform photographing in a natural position without twisting the body.

Since the supporting portion 104 supports the components such that the components extend in one direction (the direction toward the front, that is, the direction orthogonal to the A direction and the B direction), the components do not extend from the back surface (a direction opposite to the one direction (a direction toward the rear in Fig. 1). For this reason, the operator is able to bring the back surface of the supporting portion 104 close to a wall of a test room and install the ultrasonic diagnostic apparatus near the wall of the test room. Since the ultrasonic diagnostic apparatus can be installed adjacent to the wall of the test room, space for the test room is ensured.

Fig. 2 is a top view of the ultrasonic diagnostic apparatus. The details of the bed 106 and operating portion 124 of the ultrasonic diagnostic apparatus will be described with reference to Fig. 2.

The bed 106 is roughly made up of three frames. Specifically, the bed 106 is made up of a first frame 450 that supports the lumbar of the subject, a second frame 452 that supports the upper body of the subject, and a third frame 454 that supports the lower body of the subject.

The first frame 450 has a grip portion 400 and a grip portion 402 for the subject or the operator to grip. When the subject gets on the bed 106, the subject grips at least one of the grip portion 400 and the grip portion 402. The subject is able to support his or her own body with the grip portion 400 and the grip portion 402. The subject moves while gripping the grip portion 400 and the grip portion 402 such that the lumbar of the subject is located on the first frame 450. The subject is mounted near the center of the bed 106. The subject may hold the grip portion 400 and the grip portion 402 with both hands. When the subject gets on the bed 106 or gets off the bed 106, the operating portion 124 is accommodated in the panel unit 120.

When the operator wants to slide the bed 106 in the horizontal direction (A direction), the operator grips the grip portion 400 and pulls the bed 106 in a direction in which the operator wants to slide. The operator is able to slide the bed 106 in the horizontal direction. Since the grip portion 400 and the grip portion 402 are installed on the bed 106, the operability of the bed 106 improves.

The first frame 450 is fixed to the bed support member 114 shown in Fig. 1. The bed support member 114 supports the first frame 450 such that the first frame 450 is kept horizontal. The first frame 450 and the second frame 452 are separated from each other with a separation groove 420 interposed therebetween. A tilting shaft is installed at the separation groove 420. The end of the first frame 450 and the end of the second frame 452 are coupled to the tilting shaft. The first frame 450 and the second frame 452 have a structure of bending about the tilting shaft installed at the separation groove 420. The second frame 452 tilts about the tilting shaft such that the angle formed between the first frame 450 and the second frame 452 becomes an inferior angle (an angle smaller than 180 degrees). At this time, the first frame 450 does not tilt about the tilting shaft and is kept horizontal. When the second frame 452 is tilted, the bed 106 can be reclined.

Similarly, the first frame 450 and the third frame 454 are separated from each other with a separation groove 422 interposed therebetween. A tilting shaft is installed at the separation groove 422. The end of the first frame 450 and the end of the third frame 454 are coupled to the tilting shaft. The first frame 450 and the third frame 454 have a structure of bending about the tilting shaft installed at the separation groove 422. The third frame 454 tilts about the tilting shaft such that the angle formed between the first frame 450 and the third frame 454 becomes a superior angle (an angle larger than 180 degrees). At this time, the first frame 450 does not tilt about the tilting shaft and is kept horizontal. When the third frame 454 is tilted, the bed 106 can be reclined.

The bed 106 has a plurality of grooves (a groove 404, a groove 406, and a groove 408) respectively in the first frame 450, the second frame 452, and the third frame 454. The bed 106 can be separated because of the plurality of grooves. A member of the first frame 450, on which the grip portion 400 is located, can be separated because of the groove 404. When the member of the first frame 450, on which the grip portion 400 is located, is separated, the bed 106 can be formed into a U-shape. Similarly to the second frame 452 and the third frame 454, a member surrounded by the groove 406 and a member surrounded by the groove 408 can be separated, that is, part of the member of the second frame 452 and part of the member of the third frame 454 can be separated.

The operating portion 124 is installed on the panel unit 120 through the arm 132. The arm 132 is installed on the panel unit 120 through a hinge 134 (first hinge). The hinge 134 is, for example, a torque hinge. The torque hinge is a hinge that allows to rest the arm 132 at a selected position in process of opening and closing. The torque hinge has a rotating torque that allows to withstand the weight of the arm 132 and the weight of the operating portion 124. The torque hinge allows to adjust the rotating torque with an adjustment screw (not shown). In this way, the arm 132 can be turned to a predetermined angle because of the hinge 134. Therefore, as shown in Fig. 2, the operating portion 124 can be extended, and the operating portion 124 can be placed above (just above) the bed 106.

Even when the subject is mounted on the bed 124, it is possible to turn the arm 132 to extend the operating portion 124 to a predetermined angle up to which the operating portion 124 does not contact with the subject. For example, the predetermined angle can be set as 120 degrees. The arm 120 can be turned within the range of zero degrees to 120 degrees of the angle formed between the panel unit 120 and the arm 132.

Fig. 3 is a perspective view of the ultrasonic diagnostic apparatus. A configuration in which the operating portion 124 of the ultrasonic diagnostic apparatus is stored will be described with reference to Fig. 3.

As shown in Figs. 1 and 2, the arm 132 can be folded by turning the arm 132 supporting the operating portion 124. Specifically, as described above, the hinge 134 is installed between the panel unit 120 and the arm 132. Another hinge (second hinge (not shown)) is installed between the operating portion 124 and the arm 132. The operating portion 124 is installed on the arm 132 through the hinge. Specifically, the hinge is installed on the back surface of the operating portion 124. The function of the hinge (second hinge) is similar to that of the hinge 134 (first hinge), so the description is omitted.

In this way, the arm 132 is installed on the panel unit 120 through the hinge 134 (first hinge). The operating portion 124 is installed on the arm 132 through the hinge (second hinge). The arm 132 can be folded as shown in Fig. 3 with two hinges respectively installed at both ends of the arm. At this time, the arm 132 is in a state of being interposed between the panel unit 120 and the operating portion 124. In this way, the operating portion 124 can be accommodated in the panel unit 120. When the operating portion 124 is accommodated in the panel unit 120, an operating face of the operating portion 124 faces the front. Since the operating face of the operating portion 124 faces the front, even in a state where the operating portion 124 is accommodated as shown in Fig. 3, the operator is able to operate the operating portion 124 and perform photographing operation of the ultrasonic diagnostic apparatus.

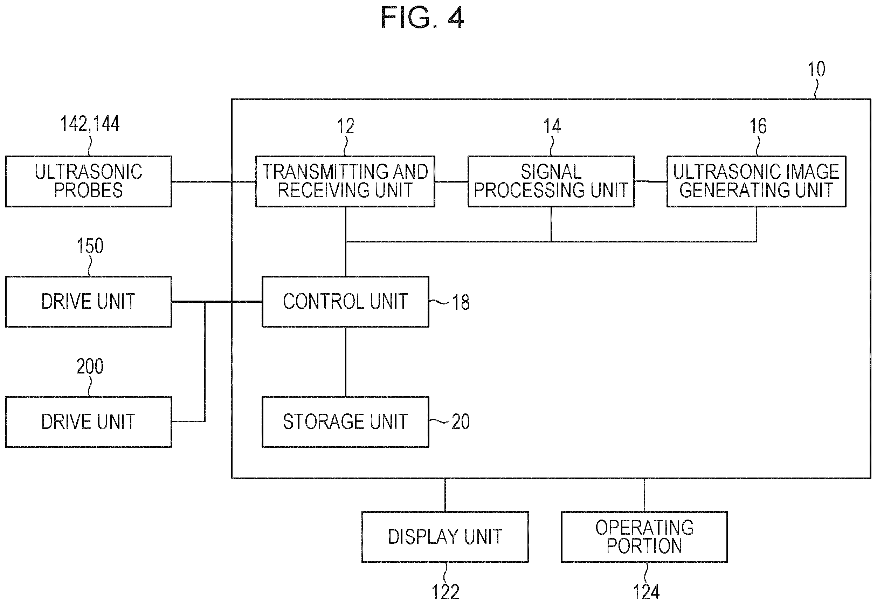

Here, the internal configuration of the ultrasonic diagnostic apparatus will be described with reference to Fig. 4. Fig. 4 is a diagram that shows an apparatus main body 10 of the ultrasonic diagnostic apparatus. The apparatus main body 10 is installed, for example, inside the supporting portion 104 or inside the panel unit 120. The apparatus main body 10 may be installed in any place of the ultrasonic diagnostic apparatus.

The apparatus main body 10 includes a transmitting and receiving unit 12 that transmits and receives ultrasonic waves to and from the ultrasonic probes 142, 144, a signal processing unit 14 that performs various signal processing using an ultrasonic signal based on a reflected wave signal received by the transmitting and receiving unit 12, an ultrasonic image generating unit 16 that generates an ultrasonic image by using signal processing data processed in the signal processing unit 14, and a control unit 18 that controls various components. The control unit 18 controls a drive unit 150 for moving the plurality of ultrasonic probes 142, 144 (described later) in the up and down direction, and a drive unit 200 for moving the panel unit 120 (the display unit 122 and the operating portion 124) and the bed 106 in the up and down direction.

Here, the ultrasonic probe 144 is assumed as the ultrasonic probe used for photographing. The transmitting and receiving unit 12 controls transmission and reception of ultrasonic waves, performed by the ultrasonic probe 144. The transmitting and receiving unit 12 has a transmitting portion, a transmission delay circuit, and the like and supplies a drive signal to the ultrasonic probe 144. The transmitting portion repeatedly generates a rate pulse with a predetermined repetition frequency (PRF). The transmission delay circuit focuses ultrasonic waves generated from the ultrasonic probe 144 and applies a delay time for determining a transmission directivity to the rate pulse generated by the transmitting portion. The transmission delay circuit is capable of controlling the transmission direction of ultrasonic waves transmitted from a vibrator by changing a delay time applied to the rate pulse.

The transmitting and receiving unit 12 has an amplifier, an A/D converter, a reception delay circuit, an adder, and the like. An ultrasonic signal is generated by performing various processing on the reflected wave signal received by the ultrasonic probe 144. The amplifier performs gain correction processing by amplifying a reflected wave signal channel by channel. The A/D converter performs A/D conversion of a gain-corrected reflected wave signal. The reception delay circuit applies a delay time to digital data to determine a reception directivity. The adder performs addition of a reflected wave signal, to which a delay time is applied, by the reception delay circuit. Through the addition performed by the adder, a reflection component in a direction according to the reception directivity of a reflected wave signal is intensified.

When the subject undergoes two-dimensional scanning, the transmitting and receiving unit 12 causes the ultrasonic probe 144 to transmit two-dimensional ultrasonic waves. Then, the transmitting and receiving unit 12 generates a two-dimensional ultrasonic signal from the two-dimensional reflected wave signal received by the ultrasonic probe 144. When the subject undergoes three-dimensional scanning, the transmitting and receiving unit 12 causes the ultrasonic probe 144 to transmit three-dimensional ultrasonic waves. Then, the transmitting and receiving unit 12 generates a three -dimensional ultrasonic signal from the three-dimensional reflected wave signal received by the ultrasonic probe 144.

The signal processing unit 14 performs various signal processing on an ultrasonic signal output from the transmitting and receiving unit 12. Specifically, the signal processing unit 14 performs signal processing, such as detection and logarithmic compression, on the ultrasonic signal. The signal processing unit 14 visualizes amplitude information of the ultrasonic signal and generates signal processing data (raster data). The ultrasonic signal output from the transmitting and receiving unit 12 undergoes bandpass filtering, and then an envelope of an output signal is detected. Then, the detected data undergoes compression by logarithmic transformation. The signal processing unit 14 outputs the processed signal processing data to the ultrasonic image generating unit 16.

The ultrasonic image generating unit 16 generates an ultrasonic image by using the signal processing data processed by the signal processing unit 14. The ultrasonic image generating unit 16 has a digital scan converter and transforms the signal processing data to data represented by rectangular coordinates. Here, the ultrasonic image generating unit 16 performs orthogonal transformation of the signal processing data (raster data) to a coordinate system (X,Y) of image data for display. Then, the ultrasonic image generating unit 16 generates an ultrasonic image (B-mode image data) in which the signal intensity is represented by the brightness of luminance. In this way, in the ultrasonic image generating unit 16, an ultrasonic image is generated. An algorithm of generating an ultrasonic image is capable of not only performing phasing addition but also performing image reconfiguration by applying a selected algorithm.

The ultrasonic image generating unit 16 is capable of generating blood flow image data by color Doppler called color flow mapping (CFM). In color Doppler, it is possible to extract movement information of blood flow by transmitting ultrasonic waves in the same direction multiple times and performing frequency analysis based on Doppler effect from the received reflected wave signal. The ultrasonic image generating unit 16 generates blood flow information such as average velocity, variance, and power as blood flow image data by using color Doppler. The ultrasonic image generating unit 16 may generate blood flow image data by power Doppler.

The operating portion 124 has a plurality of input elements and is used to input operating information of the operator. Examples of the plurality of input elements include a keyboard, a trackball, and various buttons. The operating portion 124 receives various instructions from the operator and transmits the received various instructions to the control unit 18 of the apparatus main body 10. For example, a measurement caliper moves on an ultrasonic image in accordance with the motion of the trackball or the like of the operating portion 104. The operator positions the measurement caliper in a measurement range of a measurement area. Then, the operator is able to acquire the size (for example, distance, perimeter, or area) of the measurement range by pushing a set button.

The display unit 122 displays GUI for allowing the operator to input various instructions by using the operating portion 124 or displays an ultrasonic image, blood flow image data, measurement result, and the like generated in the apparatus main body 10.

The plurality of ultrasonic probes 142, 144 is connected to the apparatus main body 10. The plurality of ultrasonic probes 142, 144 each has a plurality of vibrators and is capable of generating ultrasonic waves by actuating the plurality of vibrators. The plurality of ultrasonic probes 142, 144 receives reflected waves from the subject and converts the reflected waves to an electrical signal. The converted electrical signal is transmitted to the apparatus main body 10.

The plurality of ultrasonic probes 142, 144 each includes an acoustic matching layer provided on the front surface side (subject side) of the plurality of vibrators and used to match acoustic impedance of the subject with the plurality of vibrators, and a packing material provided on the back surface side of the plurality of vibrators and used to prevent propagation of ultrasonic waves from the plurality of vibrators toward the back surface side.

The plurality of ultrasonic probes 142, 144 is detachably connected to the frame portion 140 (the accommodation portion for the ultrasonic probes). Examples of the type of ultrasonic probe include a linear type, a sector type, a convex type, a radial type, and a three-dimensional scanning type. The operator is able to select the type of ultrasonic probe in accordance with a photographing purpose. A type of sensor applied to the ultrasonic probe is also not limited to the one using an existing bulk PZT. A capacitance probe of a type called CMUT using a fine processing technology or a probe of a type called PMUT in which a piezoelectric thin-film technology is further combined may be used.

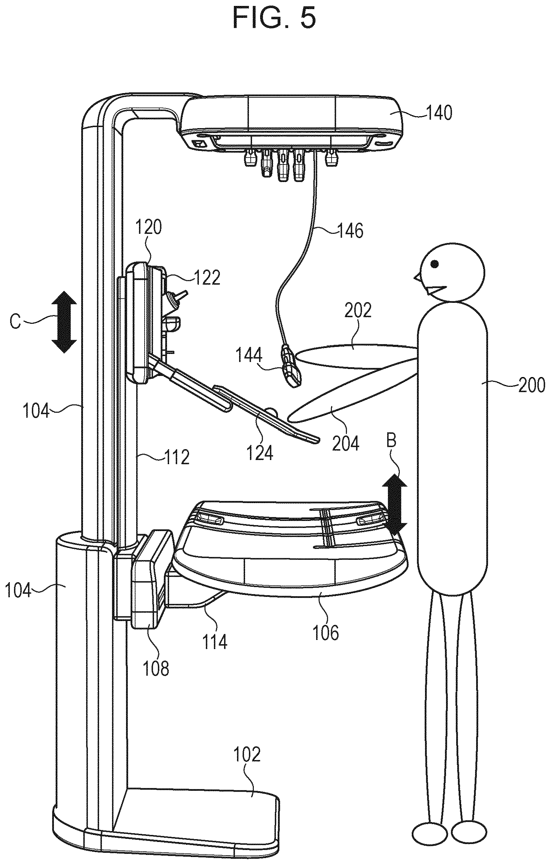

Figs. 5 to 7B are views that show up and down drive of the components in the ultrasonic diagnostic apparatus according to the present invention.

Fig. 5 is a configuration in which an operator 200 photographs the subject in a standing state. It is assumed that the operator 200 stands near the center of the bed 106 in the longitudinal direction and performs operation. The operator photographs the subject (not shown) mounted on the bed 106 by using the operating portion 124 and the ultrasonic probe 144 having lowered from the frame portion 140 (the accommodation portion for the ultrasonic probes). The ultrasonic probe 144 can transmit and receive ultrasonic waves in accordance with an electrical signal transmitted from the cable 146. At this time, a right hand 202 of the operator 200 grips the ultrasonic probe 144, and a left hand 204 of the operator 200 operates the operating portion 124.

In this way, the display unit 122, the operating portion 124, the ultrasonic probe 144, and the bed 106 can be installed toward the front of the operator 200. Therefore, the operator 200 is able to photograph in a natural position while directly facing the ultrasonic diagnostic apparatus and the subject.

The operator 200 is able to place the display unit 122 at a selected position. Since the coupling portion 112 can be moved in the up and down direction with respect to the supporting portion 104, the operator 200 is able to move the panel unit 120 in the up and down direction. The display unit 122 installed on the panel unit 120 can be moved in the up and down direction (C direction). Therefore, the display unit 122 can be placed at the same level with the level of the eyes of the operator.

The control unit 18 of the ultrasonic diagnostic apparatus is capable of automatically controlling the level of the display unit 122 and the operating portion 124 in synchronization with the body shape information of the operator 200. The ultrasonic diagnostic apparatus includes a sensor (not shown) that scans the body shape information (height and the like) of the operator 200. The body shape information of the operator 200, output from the sensor, is transmitted to the control unit 18. The control unit 18 adjusts the level of the display unit 122 and the operating portion 124 by controlling the drive unit 200 (up and down drive unit) (described later) in accordance with the body shape information of the operator. Specifically, the control unit 18 adjusts the level of the display unit 122 and the operating portion 124 such that the top end of the display unit 122 is at the level that coincides with the height of the operator 200 or such that the position of the operating portion 124 is at the level that coincides with the lumbar of the operator 200. Therefore, it is possible to reduce the leaning position of the lumbar of the operator 200.

The control unit 18 of the ultrasonic diagnostic apparatus is capable of automatically controlling the level of the display unit 122 and the operating portion 124 in synchronization with the body shape information of the operator 200, stored in advance. A storage unit 20 is capable of storing the body shape information of the operator 200. The operator 200 causes the storage unit 20 to store the body shape information in advance. For example, the operator 200 causes the storage unit 20 to store the height, line-of-sight position, and the like of the operator 200.

When the operator 200 operates the ultrasonic diagnostic apparatus, the body shape information of the operator 200 is transmitted from the storage unit 20 to the control unit 18. The control unit 18 adjusts the level of the display unit 122 and the operating portion 124 by controlling the drive unit 200 (up and down drive unit) in accordance with the body shape information of the operator 200. Specifically, the control unit 18 adjusts the level of the display unit 122 and the operating portion 124 such that the top end of the display unit 122 is placed at the level that coincides with the height of the operator 200. The control unit 18 adjusts the level of the display unit 122 and the operating portion 124 such that the screen of the display unit 122 is placed at the level that coincides with the line of sight of the operator 200 (for example, the line-of-sight direction becomes the horizontal direction).

Furthermore, the control unit 18 of the ultrasonic diagnostic apparatus is capable of automatically controlling the level of the display unit 122 and the operating portion 124 in synchronization with setting information set operator by operator. The storage unit 20 is capable of storing pieces of setting information set by a plurality of operators (for example, the level of the display unit 122 and the operating portion 124) and causes the storage unit 20 to store those pieces of setting information. Here, when the operator 200 operates the ultrasonic diagnostic apparatus, the fact that the operator 200 is operating the operating portion 124 is acquired from login information of the ultrasonic diagnostic apparatus and is transmitted to the control unit 18. The setting information of the operator 200 is transmitted from the storage unit 20 to the control unit 18 in accordance with the login information of the operator 200. The control unit 18 adjusts the level of the display unit 122 and the operating portion 124 by controlling the drive unit 200 (up and down drive unit) in accordance with the setting information of the operator 200. When another operator operates, the control unit 18 adjusts the level of the display unit 122 and the operating portion 124 by controlling the drive unit 200 (up and down drive unit) in accordance with the setting information of the another operator.

The operator 200 is able to install the bed 106 at a selected position. Since the coupling portion 112 can be moved in the up and down direction with respect to the supporting portion 104, the bed 106 can be moved in the up and down direction (B direction).

The panel unit 120 is coupled to the bed 106 through the coupling portion 112, and the distance between the panel unit 120 and the bed 106 is kept constant. The distance between the panel unit 120 and the bed 106 can be adjusted to a selected distance according to the height and usage purpose of the operator 200. For example, the operator is able to adjust the distance between the panel unit 120 and the bed 106 in a range from 50 cm to 100 cm.

When the operator moves the display unit 122 installed on the panel unit 120 in the up and down direction, the bed 106 moves in the up and down direction in synchronization with movement of the display unit 122. Similarly, when the operator moves the operating portion 124 installed on the panel unit 120 in the up and down direction, the bed 106 also moves in the up and down direction in synchronization with movement of the operating portion 124.

In other words, the display unit 122 and the bed 106 are supported on the supporting portion 104 so as to move together in the up and down direction. The display unit 122 and the bed 106 move together in a state where the distance between the display unit 122 and the bed 106 is kept constant. The operating portion 124 and the bed 106 are supported on the supporting portion 104 so as to move together in the up and down direction. The operating portion 124 and the bed 106 move together in a state where the distance between the operating portion 124 and the bed 106 is kept constant.

In other words, the supporting portion 104 is provided upright on the floor or the ceiling and supports the operating portion 124 and the bed 106 such that the operating portion 124 and the bed 106 are movable up and down. The supporting portion 104 supports the display unit 122 and the bed 106 such that the display unit 122 and the bed 106 are movable up and down. The display unit 122 and the bed 106 do not move together in the horizontal direction. Similarly, the operating portion 124 and the bed 106 do not move together in the horizontal direction.

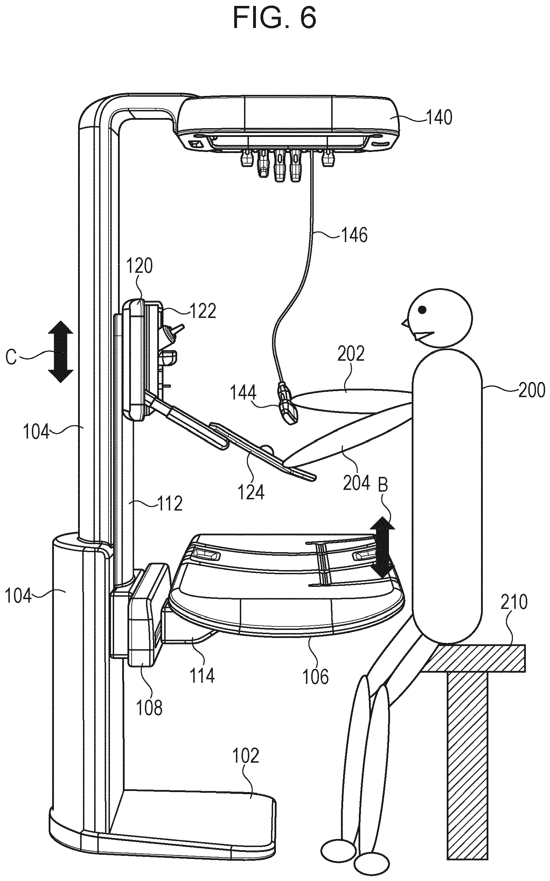

Fig. 6 is a configuration in which the operator 200 photographs the subject in a seated position. It is assumed that the operator 200 operates while being seated on a chair 210 near the center of the bed 106 in the longitudinal direction.

When the coupling portion 112 is moved downward to move the panel unit 120 (the display unit 122 and the operating portion 124) and the bed 106 downward from the configuration of the ultrasonic diagnostic apparatus shown in Fig. 5, the configuration of the ultrasonic diagnostic apparatus shown in Fig. 6 is obtained. In the configuration of the ultrasonic diagnostic apparatus shown in Fig. 6, as compared to the configuration of the ultrasonic diagnostic apparatus shown in Fig. 5, the distance between the bed 106 and the floor is short, and the distance between the bed 106 and the frame portion 140 is long.

The operator 200 photographs the subject (not shown) mounted on the bed 106 by using the operating portion 124 and the ultrasonic probe 144 having lowered from the frame portion 140 (the accommodation portion for the ultrasonic probes). The operator is able to put the knees into under the bed 106, so it is possible to avoid a lumbar twisting position.

In this way, even when the operator 200 is in a state of being seated on the chair 210, the display unit 122, the operating portion 124, the ultrasonic probe 144, and the bed 106 can be installed toward the front of the operator. Therefore, the operator 200 is able to photograph in a natural position while directly facing the subject.

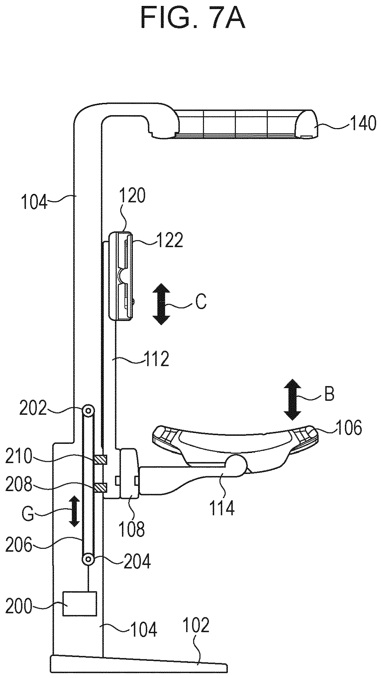

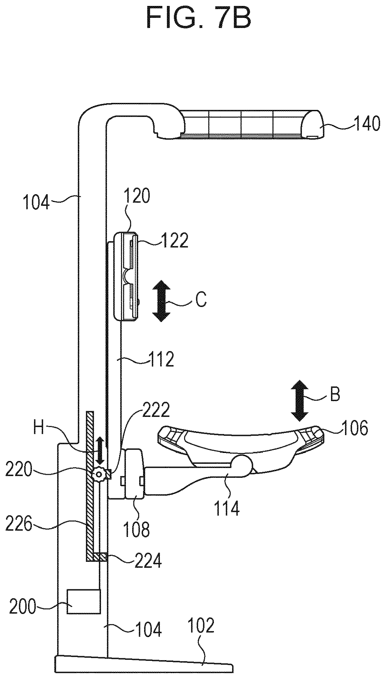

The components and up and down drive of the ultrasonic diagnostic apparatus will be described with reference to Figs. 7A and 7B. Figs. 7A and 7B are sectional views of the ultrasonic diagnostic apparatus. The sectional views of the ultrasonic diagnostic apparatus are sectional views taken along a center line (alternate long and short dashed line) extending in the up and down direction of Fig. 2.

Fig. 7A shows the configuration of an up and down drive unit (belt type) that moves the components including the bed 106, the panel unit 120 (the display unit 122 and the operating portion 124), and the like in the up and down direction.

The inside of the supporting portion 104 is hollow. A pulley 202, a pulley 204 (a plurality of pulleys), and a belt 206 are installed inside of the supporting portion 104. The pulley 202 and the pulley 204 are rotary members. The pulley 202 and the pulley 204 are installed so as to be spaced apart from each other in the longitudinal direction of the supporting portion 104. A tilting shaft of the pulley 202 and a rotating shaft of the pulley 204 both are installed on the inner wall of the supporting portion 104 and support the pulley 202 and the pulley 204 such that the pulley 202 and the pulley 204 are rotatable. The rotating shaft of the pulley 202 and the rotating shaft of the pulley 204 are parallel to a horizontal plane. The belt 206 is wound around the pulley 202 and the pulley 204 (the plurality of pulleys) in a tensioned state. The belt 206 is desirably a belt made of a material that hardly slips, a gear belt, or the like. The pulley 202 and the pulley 204 have a function of transmitting power to the belt 206.

When any one of the pulley 202 and the pulley 204 is rotated, the belt 206 slides in the up and down direction (G direction).

Belt fixing portions 208, 210 are installed on the surface of the belt 206. The belt fixing portions 208, 210 are fixed to the belt 206 and the coupling portion 112. The belt fixing portions 208, 210 and the coupling portion 112 are integrated with one another. Thus, the belt 206 and the coupling portion 112 are integrated with each other.

The coupling portion 112 is integrated with the bed 106 through the slide 108. The coupling portion 112 is integrated with the panel unit 120 (the display unit 122 and the operating portion 124). Therefore, the belt 206 is integrated with the bed 106 and the panel unit 120 (the display unit 122 and the operating portion 124).

When any one of the pulley 202 and the pulley 204 is rotated by motor drive of the drive unit 200 in the up and down drive unit to apply power to the belt 206, it is possible to slide the belt 206 in the up and down direction (G direction) and move the coupling portion 112 in the up and down direction. When the coupling portion 112 is moved in the up and down direction, the components including the bed 106, the panel unit 120 (the display unit 122 and the operating portion 124), and the like can be moved in the up and down direction (B direction and C direction).

Specifically, when the pulley 204 is rotated in the counterclockwise direction by motor drive of the drive unit 200 to rotate the belt 206 in contact with the pulley 204 in the counterclockwise direction, the belt fixing portions 208, 210 move upward. When the belt fixing portions 208, 210 move upward, the coupling portion 112 can be moved upward, and the components including the bed 106, the panel unit 120 (the display unit 122 and the operating portion 124), and the like can bee moved upward.

When the pulley 204 is rotated in the clockwise direction by motor drive of the drive unit 200 to rotate the belt 206 in contact with the pulley 204 in the clockwise direction, the belt fixing portions 208, 210 move downward. When the belt fixing portions 208, 210 move downward, the coupling portion 112 can be moved downward, and the components including the bed 106, the panel unit 120 (the display unit 122 and the operating portion 124), and the like can be moved downward.

The distance between the pulley 202 and the pulley 204 is longer than a stroke width in the up and down direction in the components including the bed 106, the panel unit 120 (the display unit 122 and the operating portion 124), and the like. A slide width of the belt 206 is equivalent to the stroke width in the up and down direction in the components including the bed 106, the panel unit 120 (the display unit 122 and the operating portion 124), and the like. If the belt 206 attempts to slide beyond the slide width, the control unit 18 locks slide of the slide 206.

In this way, the up and down drive unit (belt type) for moving the coupling portion 112 in the up and down direction is provided inside the supporting portion 104. With movement of the coupling portion 112 in the up and down direction, the components including the bed 106, the panel unit 120 (the display unit 122 and the operating portion 124), and the like can be moved in the up and down direction.

Fig. 7B shows the configuration (rack-and-pinion system) of the up and down drive unit for raising and lowering the components including the bed 106, the panel unit 120 (the display unit 122 and the operating portion 124), and the like. The inside of the supporting portion 104 is hollow. A circular gear 220 and a gear supporting portion 222 that supports the circular gear 220 such that the circular gear 220 is rotatable are installed inside the supporting portion 104. The gear supporting portion 222 is fixed to the coupling portion 112, and the gear supporting portion 222 and the coupling portion 112 are integrated with each other.

The coupling portion 112 is integrated with the bed 106 through the slide 108. The coupling portion 112 is integrated with the panel unit 120 (the display unit 122 and the operating portion 124). Therefore, the circular gear 220 and the gear supporting portion 222 are integrated with the bed 106 and the panel unit 120 (the display unit 122 and the operating portion 124).

The gear supporting portion 222 supports the circular gear 220 such that a rotating shaft of the circular gear 220 is parallel to the horizontal plane. The circular gear 220 can be rotated by motor drive. The drive unit 200 for rotating the circular gear 220 with motor drive is provided inside the supporting portion 104.

A plate-shaped rod member 226 and a rod member supporting portion 224 that supports the rod member 226 are installed inside the supporting portion 104. The rod member supporting portion 224 inscribes the supporting portion 104, and the rod member supporting portion 224 is fixed to the supporting portion 104. The plate-shaped rod member 226 is installed along the longitudinal direction (up and down direction) of the supporting portion 104. The longitudinal direction of the supporting portion 104 is a direction orthogonal to the horizontal plane.

The surface of the rod member 226 has an uneven shape that matches the gear shape of the circular gear 220.

The circular gear 220 and the rod member 226 are in mesh with each other. When rotational force is applied to the circular gear 220 by the drive unit 200, the circular gear 220 rolls on the surface of the rod member 226, and the coupling portion 112 can be moved in the up and down direction (H direction). In other words, the circular gear 220 can be moved in the up and down direction by the drive unit 200. When the coupling portion 112 is moved in the up and down direction, the components including the bed 106, the panel unit 120 (the display unit 122 and the operating portion 124), and the like can be moved in the up and down direction (B direction and C direction).

Specifically, when the circular gear 220 is rotated in the clockwise direction by the drive unit 200 in the up and down drive unit, the circular gear 220 rolls on the rod member 226 and moves downward along the longitudinal direction of the rod member 226. When the circular gear 220 moves downward, the coupling portion 112 can be moved downward, and the components including the bed 106, the panel unit 120 (the display unit 122 and the operating portion 124), and the like can bee moved downward.

When the circular gear 220 is rotated in the counterclockwise direction by the drive unit 200, the circular gear 220 rolls on the rod member 226 and moves upward along the longitudinal direction of the rod member 226. When the circular gear 220 moves upward, the coupling portion 112 can be moved upward, and the components including the bed 106, the panel unit 120 (the display unit 122 and the operating portion 124), and the like can bee moved upward.

As for the type of up and down drive according to the present invention, the example of the belt system and the example of the rack-and-pinion system have been described above, and other configurations such as a gear system and a combination of a cam follower and a guide rail may be used.

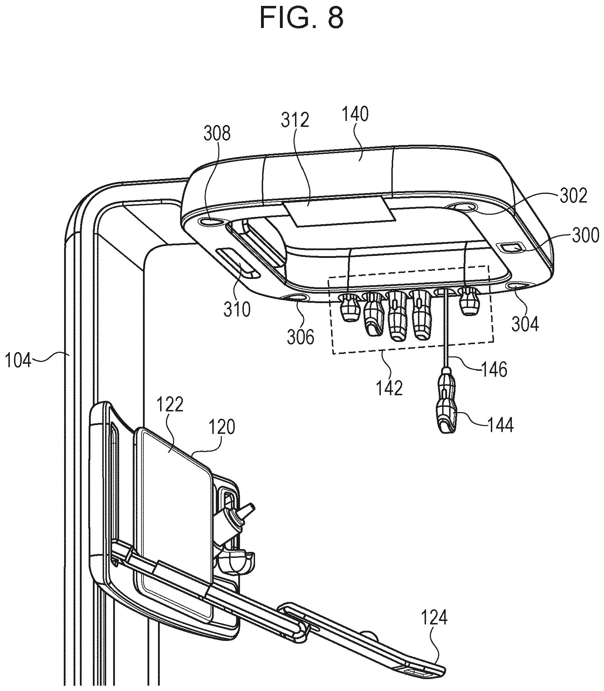

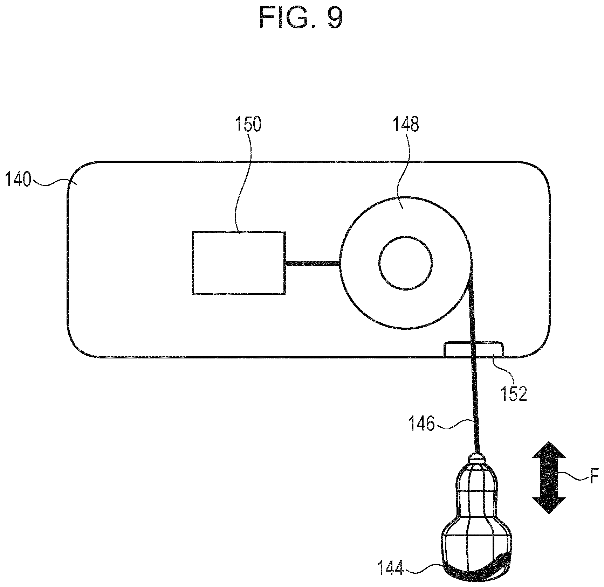

Figs. 8 and 9 are views that show the configuration of the frame portion 140 in the ultrasonic diagnostic apparatus according to the present invention.

As shown in Fig. 8, the supporting portion 104 supports the frame portion 140 at the topmost part. The frame portion 140 does not move in the up and down direction. The frame portion 140 accommodates the ultrasonic probes 142, 144. The frame portion 140 has an emergency stop button 300, projection units 302, 304, cameras 306, 308, and a sensor 310 in addition to the accommodation portion for the ultrasonic probes.

The emergency stop button 300 is a button for stopping the operation of the components of the ultrasonic diagnostic apparatus. When the components including the bed 106, the panel unit 120 (the display unit 122 and the operating portion 124), and the like are moving in the up and down direction with the drive unit 200, there can be a risk that the subject mounted on the bed 106 contacts with the components (for example, the display unit 122 and the operating portion 124) of the ultrasonic diagnostic apparatus.

At this time, when the operator pushes the emergency stop button 300, the operation of the components of the ultrasonic diagnostic apparatus is stopped. Specifically, the emergency stop button 300 is connected to the control unit 18. When the emergency stop button 300 is pushed, the fact that the emergency stop button 300 is pushed (stop signal) is transmitted to the control unit 18. The control unit 18 stops motor drive of the drive unit 200 and stops movement of the coupling portion 112 in the up and down direction. Therefore, movement of the components including the bed 106, the panel unit 120 (the display unit 122 and the operating portion 124), and the like is stopped.

In this way, it is possible to avoid, with the emergency stop button 300, a risk that the components of the ultrasonic diagnostic apparatus contact with the subject.

Control of the drive unit 200 has been described as an example of stopping the operation of the components of the ultrasonic diagnostic apparatus. Alternatively, when the operator pushes the emergency stop button 300, the control unit 18 may stop transmission and reception of ultrasonic waves in the ultrasonic probe 144.

The frame portion 140 has the camera 306 and the camera 308 for mainly photographing the subject mounted on the bed 106. The control unit 18 is capable of displaying images photographed by the camera 306 and the camera 308 on the display unit 122. The camera 306 and the camera 308 each may be a depth camera that acquires depth information (up and down direction) of the subject mounted on the bed 106.

The camera 306 and the camera 308 are arranged parallel to each other. The depth information of the subject can be measured by the control unit 18 performing image processing on images acquired from two cameras. For example, in a stereo camera system, the depth information of the subject is measured from parallax information of the camera 306 and the camera 308. In a ToF (Time of Flight) camera system, near infrared rays are emitted from any one of the camera 306 and the camera 308 to the subject, and a time taken until reflected waves reflected from the subject reach is measured. Thus, the depth information of the subject is measured. The control unit 18 is capable of acquiring three-dimensional information including the depth information of the subject with the camera 306 and the camera 308. When the depth information of the subject is grasped, the control unit 18 is capable of setting the drawn-out amount (length) of the cable 146 of the ultrasonic probe 144 used for photographing.

The control unit 18 is capable of acquiring various pieces of information of the subject by camera recognition technology. The control unit 18 is capable of acquiring, for example, the body constitution of the subject, the position (photographing area) of an organ with which the ultrasonic probe 144 contacts, and the like. The control unit 18 is capable of associating an ultrasonic image generated by the ultrasonic image generating unit 16 with the depth information of the subject, the body constitution of the subject, and the photographed area. The storage unit 20 is capable of storing an ultrasonic image, depth information of the subject, a body constitution of the subject, and a photographed area in association with one another.

The projection unit 302 and the projection unit 304 each project a projection image on the bed 106 or the subject mounted on the bed 106.

The projection unit 302 and the projection unit 304 each have, for example, a lamp, a lens, and the like of a light source. The projection unit 302 and the projection unit 304 each decompose the lamp of the light source into red, green, and blue that are three primary colors of light. Then, the projection unit 302 and the projection unit 304 each generate a video of each color with a transmissive panel and project the video from the lens.

Images projected from the projection unit 302 and the projection unit 304 may include positional information of the ultrasonic probe 144 having photographed last time. Specifically, the camera 306 and the camera 308 are connected to the control unit 18. The camera 306 and the camera 308 each photograph the ultrasonic probe 144 in contact with the subject, and the control unit 18 analyzes the images acquired from the camera 306 and the camera 30. The control unit 18 acquires the positional information of the ultrasonic probe 144 from the analyzed images. The storage unit 20 stores the positional information of the ultrasonic probe 144. The projection unit 302 and the projection unit 304 acquire the positional information of the ultrasonic probe 144, stored in the storage unit 20, and project the positional information of the ultrasonic probe 144 on the subject mounted on the bed 106.n The operator is able to check the positional information of the ultrasonic probe 144 photographed last time from the projected image and photograph by bringing the ultrasonic probe 144 into contact at the same position. Thus, the operator is able to check a temporal change from the last and current pieces of ultrasonic image data.

Two projection unit 302 and projection unit 304 are installed in the frame portion 140. When the images projected from the projection unit 302 and the projection unit 304 are synthesized, the image projected on the bed 106 or the subject mounted on the bed 106 can be displayed in a large size.

A content projected from the projection unit 302 and a content projected from the projection unit 304 may be differentiated from each other. For example, an image may be projected from one projection unit, and explanatory text may be projected from the other projection unit.

The projection unit 302 and the projection unit 304 may function as an illumination portion that illuminates an area on the bed 106. The test room in which the ultrasonic diagnostic apparatus is installed is dark, and the subject may be difficult to recognize the ultrasonic diagnostic apparatus. When the projection unit 302 and the projection unit 304 illuminate the bed 106, the subject is able to easily recognize the ultrasonic diagnostic apparatus. In addition, when the projection unit 302 and the projection unit 304 illuminate the bed 106, the floor near the ultrasonic diagnostic apparatus becomes bright. This may reduce stumbling of the operator or the subject.

It is also possible to set illumination light by combining red, green, and blue in the lamp of the light source of the projection unit 302 and the lamp of the light source of the projection unit 304. The projection unit 302 and the projection unit 304 each may also have a function of narrowing an irradiation field such that illumination light does not strike the face of the subject.

The sensor 310 is, for example, a motion sensor and is capable of detecting the presence or absence of an operator near the ultrasonic diagnostic apparatus and the presence or absence of a subject mounted on the bed 106. The sensor 310 and the control unit 18 are connected to each other, and information on the presence or absence of an operator and/or a subject is transmitted to the control unit 18. The control unit 18 is capable of executing various control in accordance with the presence or absence of an operator and/or a subject. When, for example, the operator approaches the ultrasonic diagnostic apparatus and the subject, the control unit 18 starts up the projection unit 302 and the projection unit 304 as the illumination portion. When the subject is mounted on the bed 106, the control unit 18 limits up and down movements of the above-described up and down drive unit.

As shown in Fig. 8, the accommodation portion for accommodating the plurality of ultrasonic probes 142, 144 is provided in the frame portion 140. The accommodation portion for accommodating the ultrasonic probes in the frame portion 140 is installed above the bed 106. In other words, the accommodation portion for accommodating the ultrasonic probes is installed at a position higher than the position where the bed 106 is installed. The accommodation portion for accommodating the ultrasonic probes is provided above the operating portion 124 or the display unit 122. In other words, the accommodation portion for accommodating the ultrasonic probes is provided at a position higher than the position where the operating portion 124 or the display unit 122 is installed.

The plurality of ultrasonic probes 142, 144 is arranged in the frame portion 140 and accommodated. The plurality of ultrasonic probes 142, 144 is arranged in a direction parallel to a transverse direction of the bed 106 and accommodated. The plurality of ultrasonic probes 142, 144 is accommodated so as to be arranged at equal intervals and not to contact with each other. Since the plurality of ultrasonic probes 142, 144 is arranged at one side of the frame portion 140, the cables of the respective ultrasonic probes are not entangled even when the ultrasonic probe 144 used for photographing is lowered from the frame portion 140. Since the ultrasonic probe 144 is suspended from the frame portion 140, the cable 146 of the ultrasonic probe 144 is installed above the ultrasonic probe 144. In other words, the cable 146 is installed at a position higher than the position where the ultrasonic probe 144 is installed. The ultrasonic probe 144 contacts with the subject; however, when there is no slack in the cable 146 installed above the ultrasonic probe 144, the cable 146 does not contact with the subject. Thus, the cable 146 can keep its sanitary state.

Fig. 9 is a view that shows the cross section of the frame portion 140 and is a view that shows the configuration of the accommodation portion for the ultrasonic probes. Here, an accommodation configuration related to the ultrasonic probe 144 is shown, and a similar accommodation configuration is also used for the other plurality of ultrasonic probes 142.

As shown in Fig. 9, a hole 152 is formed at the bottom surface of the frame portion 140. The cable 146 of the ultrasonic probe 144 is disposed through the hole 152. The frame portion 140 has a take-up portion 148 for taking up the cable 146 of the ultrasonic probe 144 and the drive unit 150 for actuating the take-up portion 148.

The cable 146 is wound around the take-up portion 148. A rotating shaft of the take-up portion 148 is installed on the inner wall of the frame portion 140 and supports the take-up portion 148 such that the take-up portion 148 is rotatable. The rotating shaft of the take-up portion 148 is parallel to the horizontal plane.

The take-up portion 148 can be rotated by motor drive of the drive unit 150 to move the cable 146 in the up and down direction (F direction). Therefore, the ultrasonic probe 144 can be moved in the up and down direction.

When the drive unit 150 rotates the take-up portion 148 in the clockwise direction, the cable 146 can be drawn out to lower the ultrasonic probe 144 downward. The operator is able to photograph with the ultrasonic probe 144 lowered downward.

A drawn-out rotation amount (in the clockwise direction) of the take-up portion 148 corresponds to the length by which the cable 146 is drawn out. A rotation amount of the take-up portion 148 may be set in advance in the drive unit 150 The control unit 18 can set in advance the drawn-out rotation amount (in the clockwise direction) of the take-up portion 148 for the drive unit 150 such that the length of the cable 146 from the frame portion 140 becomes a predetermined length (for example, 100 cm).

When the drive unit 150 rotates the take-up portion 148 in the counterclockwise direction, the cable 146 can be returned to move the ultrasonic probe 144 upward. A return rotation amount (counterclockwise direction) of the take-up portion 148 is equivalent to the drawn out rotation amount (clockwise direction) of the take-up portion 148.

In this way, the operator is able to accommodate the ultrasonic probe 144.

The operator is able to select the ultrasonic probe used for photographing from among the plurality of ultrasonic probes 142 accommodated in the frame portion 140 (the accommodation portion for the ultrasonic probes). When the ultrasonic probe used for photographing is selected by the operator, the control unit 18 controls the drive unit 150 such that the take-up portion 148 corresponding to the selected ultrasonic probe 144 is rotated in the clockwise direction. The selected ultrasonic probe 144 lowers from the frame portion 140.

The control unit 18 can be set such the ultrasonic probe 144 that coincides with a test area of the subject or that coincides with a photographing condition lowers from the frame portion 140. When a desired area (first area) in the subject is photographed, the control unit 18 controls the drive unit 150 for moving up and down the ultrasonic probe capable of photographing a desired area (first area). When a desired area (second area) in the subject is photographed, the control unit 18 controls the drive unit 150 for up and down movement of the ultrasonic probe capable of photographing a desired area (second area). When the subject is photographed under a desired photographing condition (first photographing condition), the control unit 18 controls the drive unit 150 that moves up and down the ultrasonic probe capable of photographing under the desired photographing condition (first photographing condition). When the subject is photographed under a desired photographing condition (second photographing condition), the control unit 18 controls the drive unit 150 that moves up and down the ultrasonic probe capable of photographing under the desired photographing condition (second photographing condition).

When, for example, the carotid artery of the subject is photographed, the control unit 18 controls the drive unit 150 that moves up and down the linear ultrasonic probe to rotate the take-up portion 148 in the clockwise direction. In this way, the cable of the linear ultrasonic probe is drawn out, and the linear ultrasonic probe lowers from the frame portion 140. At this time, it is also possible to inform the operator that the linear ultrasonic probe is selected, by illuminating the illumination portion (LED) installed in the linear ultrasonic probe.

When the abdomen of the subject is photographed, the control unit 18 controls the drive unit 150 that moves up and down a convex ultrasonic probe to rotate the take-up portion 148 in the clockwise direction. In this way, the cable of the convex ultrasonic probe is drawn out, and the convex ultrasonic probe lowers from the frame portion 140. At this time, it is also possible to inform the operator that the convex ultrasonic probe is selected, by illuminating the illumination portion (LED) installed in the convex ultrasonic probe.

When the heart of the subject is photographed, the control unit 18 controls the drive unit 150 that moves up and down a sector ultrasonic probe to rotate the take-up portion 148 in the clockwise direction. In this way, the cable of the sector ultrasonic probe is drawn out, and the sector ultrasonic probe is lowered from the frame portion 140. At this time, it is also possible to inform the operator that the sector ultrasonic probe is selected, by illuminating the illumination portion (LED) installed in the sector ultrasonic probe.

The ultrasonic probe 144 having lowered from the frame portion 140 may be transmitting and receiving ultrasonic waves so as to be capable of immediately photographing the subject. The operator is able to photograph the subject mounted on the bed 106 by using the ultrasonic probe 144 having lowered from the frame portion 140.

The take-up portion 148 may use a spring (not shown) installed inside the take-up portion 148. When the cable 146 is drawn out, a drum rotates, and the spring is taken up. The ultrasonic probe 144 and the cable 146 are drawn out from the frame portion 140.

The spring has properties to return to an original state. The cable 146 returns to the hole 152 and is taken up by the take-up portion 148; however, rotation of the drum is suppressed by a brake (not shown), and return of the cable 146 is suppressed. In a state where the ultrasonic probe 144 and the cable 146 are drawn out from the frame portion 140, the operator photographs the subject with the ultrasonic probe 144.

After the subject is photographed, the operator releases the brake. When the brake is released, the spring attempts to return to an original state and, as a result, the drum rotates, so the cable 146 is taken up. In this way, the ultrasonic probe 144 and the cable 146 are accommodated in the frame portion 140.

When there is no desired ultrasonic probe among the plurality of ultrasonic probes 142 accommodated in the frame portion 140 (the accommodation portion for the ultrasonic probes), it is possible to replace the ultrasonic probes. Specifically, a connector (not shown) is provided at the end of the cable 146, and the ultrasonic probe 144 and the cable 146 can be separated from each other.

The connector is a member for connecting the ultrasonic probe 144 to the ultrasonic diagnostic apparatus. The connector has a terminal for engagement with the ultrasonic probe 144. When the ultrasonic probe 144 and the cable 146 are connected by the connector, the ultrasonic probe 144 and the transmitting and receiving unit 12 in the apparatus main body 10 can be connected to each other. When another ultrasonic probe and the cable 146 are connected to each other by the connector, the another ultrasonic probe and the transmitting and receiving unit 12 in the apparatus main body 10 can be connected to each other.

Here, the ultrasonic probe 144 and the cable 146 have been described; however, another ultrasonic probe and a cable also have a similar configuration. In this way, when the ultrasonic probe is replaced, various ultrasonic probes, such as a radial probe and an endoscope probe, not shown in Fig. 8, may be installed.

Each of the plurality of ultrasonic probes 142, 144 has a cable and alternatively may be a wireless ultrasonic probe. The wireless ultrasonic probe may have a wireless transmitting portion, a wireless receiving portion, a battery, and the like. Wireless communication between the wireless ultrasonic probe and the apparatus main body 10 can be performed by using the wireless transmitting portion and the wireless receiving portion.

The wireless ultrasonic probe can be fitted to the hole 152 of the frame portion 140.

The frame portion 140 has a function of charging the battery of the wireless ultrasonic probe.

A display unit 312 for displaying an ultrasonic image may be installed in the frame portion 140. The display unit 312 is detachably mounted to the frame portion 140. The display unit 312 is connected to the apparatus main body 10. The display unit 312 is capable of displaying an ultrasonic image generated by the ultrasonic image generating unit 16. A display surface of the display unit 312 faces downward and faces toward the subject mounted on the bed 106. The subject is able to watch the display unit 312 when placed on his or her back. In other words, the subject is able to watch the display unit 312 in a natural state without changing his or her position. The subject is able to check a disease condition, a situation of a fetus, or the like from ultrasonic image data. The operator is able to explain a disease condition, a situation of a fetus, or the like in a state where the subject is mounted on the bed 106.

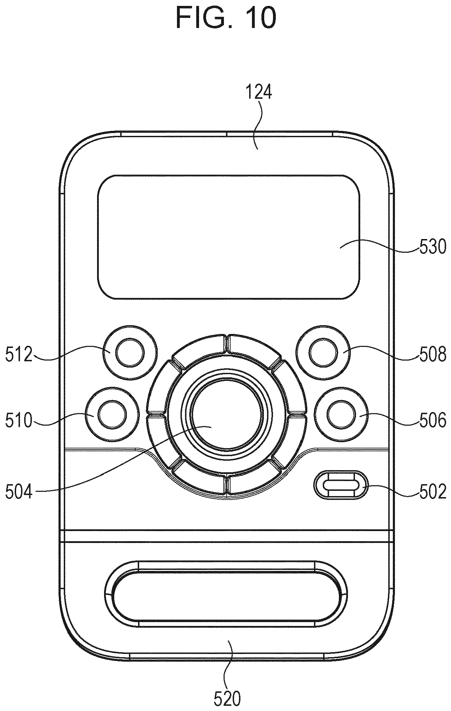

Fig. 10 is a view that shows the configuration of the operating portion 124 in the ultrasonic diagnostic apparatus according to the present invention.

The operating portion 124 has a freeze button 502 for freezing an ultrasonic image displayed on the display unit 122. The freeze button 502 of the operating portion 124 is a button for freezing an ultrasonic image displayed in real time when the ultrasonic image is stored. When the operator pushes the freeze button 502 in a state where the ultrasonic probe 144 is not moved, an ultrasonic image displayed in real time on the display unit 122 can be frozen. Data of the frozen ultrasonic image can be stored in the storage unit 20.

The operating portion 124 has a trackball 504. When the operator operates the trackball 504, it is possible to move the measurement caliper or move various commands.

The operating portion 124 has switches 506 to 510 in a circular arc shape about the trackball 504. The switches 506 to 510 include a B mode switch for selecting a B mode as ultrasonic photographing, a CFM mode switch for selecting a CFM mode, a Doppler mode switch for selecting a Doppler mode, an M mode switch for selecting an M mode, and the like. When a button is selected from among the switches 506 to 510, illumination is performed with an LED or the like. The operator can grasp the fact that a button is selected.

The operating portion 124 has a handle 520 for the operator to grasp. The operator is able to move the operating portion 124 by grasping the handle 520. For example, as shown in Fig. 1, the operating portion 124 can be drawn out from the panel unit 120 and, as shown in Fig. 3, can be accommodated in the panel unit 120.

The handle 520 is formed such that the inclination is formed upward with respect to the surface of the upper part of the operating portion 124 and also has a function of a palm rest. The operator is able to operate the freeze button 502, the trackball 504, and the switches 506 to 510 while putting the palm on the handle 520.

The operating portion 124 has a screen 530. The screen 530 displays a selected mode, information of the subject, and the like by the switches 506 to 510.

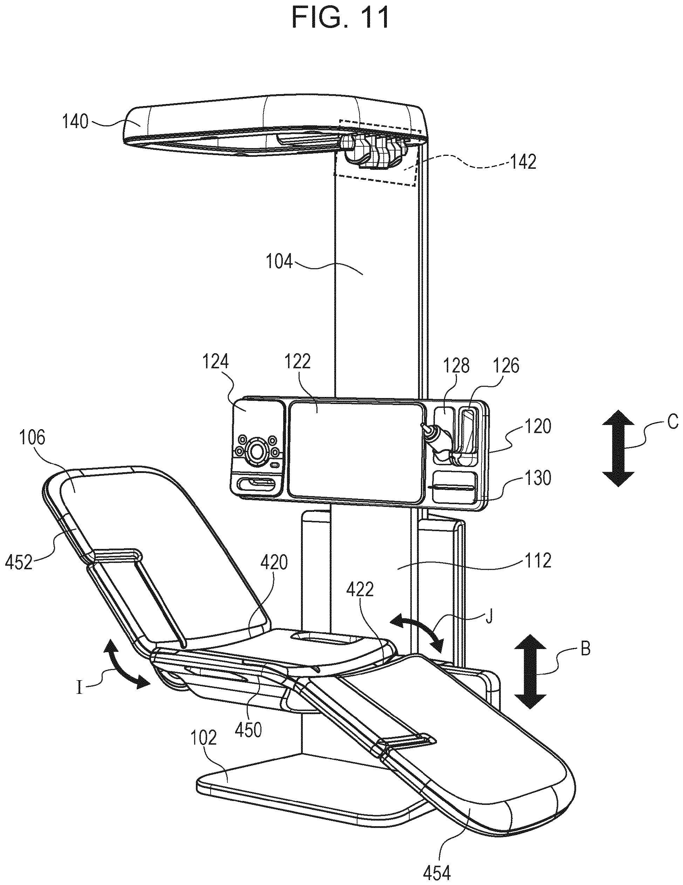

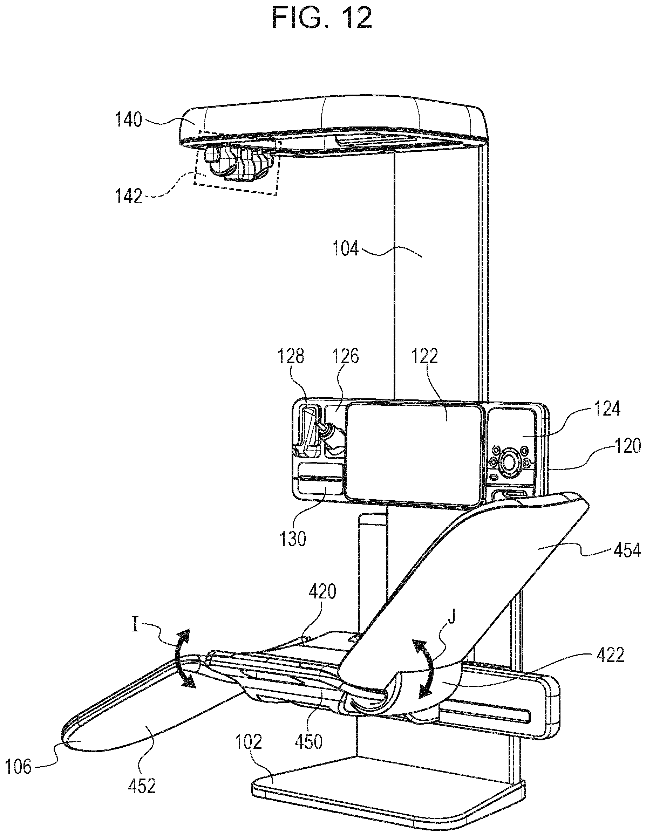

Figs. 11 and 12 are views that show a modification of the bed 106 in the ultrasonic diagnostic apparatus according to the present invention.

As shown in Fig. 11, the bed 106 is made up of the first frame 450 that supports the lumbar of the subject, the second frame 452 that supports the upper body of the subject, and the third frame 454 that supports the lower body of the subject.

The tilting shaft is installed between the first frame 450 and the second frame 452. In other words, the tilting shaft is installed in the separation groove 420. The tilting shaft between the first frame 450 and the second frame 452 is parallel to the horizontal plane and parallel to the transverse direction of the bed 106.

The drive unit is installed inside (separation groove 420) of the bed 106. The second frame 452 can be rotated in a predetermined rotation direction (I direction) about the tilting shaft with motor drive of the drive unit. The operator is able to incline the second frame 452 upward by rotating the second frame 452. When the second frame 452 is inclined upward, the upper body of the subject can be raised.

The tilting shaft is installed between the first frame 450 and the third frame 454. In other words, the tilting shaft is installed in the separation groove 422. The tilting shaft between the first frame 450 and the third frame 454 is parallel to the horizontal plane and parallel to the transverse direction of the bed 106.

The drive unit is installed inside (separation groove 422) of the bed 106. The third frame 454 can be rotated in a predetermined rotation direction (J direction) about the tilting shaft with motor drive of the drive unit. The operator is able to incline the third frame 454 downward by rotating the third frame 454 about the tilting shaft. When the third frame 454 is inclined downward, the lower body of the subject can be lowered.

As shown in Fig. 11, the bed 106 can be reclined into a shape like a chair. The operator is able to photograph the subject by using the ultrasonic probe in a state where the bed 106 is reclined.

As shown in Fig. 11, the subject is able to get on the bed 106 in a state where the bed 106 is in a reclined state (in a state with a backrest). Then, the state of the ultrasonic diagnostic apparatus shown in Fig. 11 is changed into the state of the ultrasonic diagnostic apparatus shown in Fig. 1, and the bed 106 can be set to a horizontal state. Specifically, the operator rotates the second frame 452 to set the second frame 452 in a horizontal state and rotates the third frame 454 to set the third frame 454 in a horizontal state. In this way, the bed 106 can be set to a horizontal state, and the operator is able to photograph the subject in a state where the bed 106 is horizontal. Furthermore, when the operator moves the coupling portion 112 in the up and down direction as described above, the components including the bed 106, the panel unit 120 (the display unit 122 and the operating portion 124), and the like can be moved in the up and down direction (B direction and C direction) .

When the subject gets off the bed 106, the operator sets the bed 106 to a reclined state (in a state with a backrest) as shown in Fig. 11. The subject is able to easily get on and off the bed 106.

Fig. 12 is a view that shows a configuration in which a reclining direction of the bed 106 shown in Fig. 11 is different.

In the configuration of the bed 106 shown in Fig. 11, the second frame 452 supports the upper body of the subject; whereas, in the configuration of the bed 106 shown in Fig. 12, the second frame 452 supports the lower body of the subject. The third frame 454 supports the lower body of the subject; whereas, in the configuration of the bed 106 shown in Fig. 12, the third frame 454 supports the upper body of the subject.

The second frame 452 can be rotated in a predetermined rotation direction (I direction) with motor drive of the drive unit. As shown in Fig. 12, the operator is able to incline the second frame 452 downward by rotating the second frame 452. When the second frame 452 is inclined downward, the lower body of the subject can be lowered.

The third frame 454 can be rotated in a predetermined rotation direction (J direction) with motor drive of the drive unit. As shown in Fig. 12, the operator is able to incline the third frame 454 upward by rotating the third frame 454. When the third frame 454 is inclined upward, the upper body of the subject can be raised.

In the configuration of the panel unit 120 shown in Fig. 11, the operating portion 124 for the operator to operate is installed at the left side, and the probe holder 126, the bottle accommodation portion 128, and the tissue paper box 130 are installed at the right side.

The operating portion 124 is detachably mounted to the panel unit 120. As shown in Fig. 12, the operating portion 124 can be installed at the right side of the panel unit 120. Similarly, the probe holder 126, the bottle accommodation portion 128, and the tissue paper box 130 are detachably attachable to the panel unit 120. As shown in Fig. 12, the probe holder 126, the bottle accommodation portion 128, and the tissue paper box 130 can be installed at the left side of the panel unit 120.

In the configuration of the frame portion 140 shown in Fig. 11, the plurality of ultrasonic probes 142 is accommodated at the right side. The accommodation portion for the ultrasonic probes is detachably mounted to the frame portion 140.

As shown in Fig. 12, the accommodation portion for the ultrasonic probes can be installed at the left side of the frame portion 140.

As described above, an ultrasonic diagnostic apparatus according to the present invention includes an ultrasonic probe 142, 144 that transmits and receives ultrasonic waves to and from a subject, a display unit 122 that displays an ultrasonic image based on the ultrasonic waves transmitted and received by using the ultrasonic probe 142, 144, an operating portion 124 that has a plurality of input elements and that is used to input operating information of an operator, a bed 106 for mounting the subject, and a supporting portion 104 that is provided upright on a floor or a ceiling and that supports the operating portion 124 and the bed 106. Then, the operating portion 124 is installed above the bed 106. The operating portion 124 is installed at a position higher than a position where the bed 106 is installed.

Since the operating portion 124 is installed above the bed 106, the operator is able to directly face the subject mounted on the bed 106 and photograph in a natural position and also operate the operating portion 124.

The supporting portion 104 in the ultrasonic diagnostic apparatus according to the present invention supports the operating portion 124 and the bed 106 such that the operating portion 124 and the bed 106 are movable up and down and supports the display unit 122 and the bed 106 such that the display unit 122 and the bed 106 are movable up and down.

The ultrasonic diagnostic apparatus according to the present invention includes an accommodation portion (frame portion 140) that accommodates a plurality of the ultrasonic probes. The supporting portion 104 supports the accommodation portion (frame portion 140).

A computer program that implements control functions (including various functions) of the ultrasonic diagnostic apparatus according to the present invention is supplied to a computer (control unit 18) of the ultrasonic diagnostic apparatus via a network or a storage medium (not shown) to cause the computer to run the computer program.

The computer program is a program for implementing the functions of the ultrasonic diagnostic apparatus by the computer (control unit 18). A storage medium stores the computer program.

Embodiments of the present invention are not limited to the above-described embodiments. Various changes or modifications are applicable without departing from the spirit and scope of the present invention. Therefore, the following claims are attached to show the scope of the present invention.

This application claims the benefit of Japanese Patent Application No. 2021-157068 filed September 27, 2021, which is hereby incorporated by reference herein in its entirety.

## Claims

1. An ultrasonic diagnostic apparatus comprising:
an ultrasonic probe that transmits and receives ultrasonic waves to and from a subject;
an accommodation portion that accommodates the ultrasonic probe;
a display unit that displays an ultrasonic image based on the ultrasonic waves transmitted and received by using the ultrasonic probe;
an operating portion that has a plurality of input elements and that is used to input operating information of an operator;
a bed for mounting the subject; and
a supporting portion that is installed on a floor or a ceiling and that supports the operating portion, the bed, and the accommodation portion.

2. The ultrasonic diagnostic apparatus according to Claim 1, wherein the accommodation portion is installed above the bed.

3. The ultrasonic diagnostic apparatus according to Claim 1, wherein the operating portion is installed above the bed, and the accommodation portion is installed above the operating portion.

4. The ultrasonic diagnostic apparatus according to Claim 1, wherein the accommodation portion accommodates a plurality of the ultrasonic probes such that the ultrasonic probes are arranged at equal intervals.

5. The ultrasonic diagnostic apparatus according to Claim 1, wherein the supporting portion supports a frame portion, and the accommodation portion is installed in the frame portion.

6. The ultrasonic diagnostic apparatus according to Claim 5, wherein the supporting portion supports the frame portion at a topmost part.

7. The ultrasonic diagnostic apparatus according to Claim 5, wherein, when a cable of the ultrasonic probe is drawn out from the accommodation portion, the accommodation portion is installed in the frame portion such that the ultrasonic probe and the operating portion do not interfere with each other.

8. The ultrasonic diagnostic apparatus according to Claim 1, wherein the accommodation portion is not installed over the operating portion.

9. The ultrasonic diagnostic apparatus according to Claim 5, wherein the frame portion includes a take-up portion that takes up a cable of the ultrasonic probe and a drive unit that actuates the take-up portion.

10. The ultrasonic diagnostic apparatus according to Claim 9, further comprising a control unit that controls a drive unit that moves the ultrasonic probe up and down.

11. The ultrasonic diagnostic apparatus according to Claim 10, wherein, when a first area in the subject is photographed, the control unit controls the drive unit that moves up and down the ultrasonic probe capable of photographing the first area.

12. The ultrasonic diagnostic apparatus according to Claim 10, wherein, when the subject is photographed under a first photographing condition, the control unit controls the drive unit that moves up and down the ultrasonic probe capable of photographing the subject under the first photographing condition.

13. The ultrasonic diagnostic apparatus according to Claim 5, wherein the frame portion has an emergency stop button for stopping an operation of a component of the ultrasonic diagnostic apparatus.

14. The ultrasonic diagnostic apparatus according to Claim 5, wherein the frame portion includes a camera that photographs the subject mounted on the bed.

15. The ultrasonic diagnostic apparatus according to Claim 14, wherein the camera has a function of acquiring depth information of the subject.

16. The ultrasonic diagnostic apparatus according to Claim 5, wherein the frame portion includes a projection unit that projects a projection image onto the bed or the subject mounted on the bed.

17. The ultrasonic diagnostic apparatus according to Claim 16, further comprising a control unit that analyzes an image acquired from a camera photographing the subject mounted on the bed and that acquires positional information of the ultrasonic probe, and a storage unit that stores the positional information of the ultrasonic probe, wherein the projection unit projects the positional information of the ultrasonic probe, stored in the storage unit, onto the subject mounted on the bed.

18. The ultrasonic diagnostic apparatus according to Claim 5, wherein the frame portion includes a motion sensor that detects presence or absence of the subject.

19. The ultrasonic diagnostic apparatus according to Claim 5, wherein the display unit is installed in the frame portion.
